# EUROPÄISCHE PATENTSCHRIFT

(11) **EP 0 757 041 B1**
(45) Veröffentlichungstag und Bekanntmachung des Hinweises auf die Patenterteilung: **03.05.2000**
(21) Anmeldenummer: 96111590.4
(22) Anmeldetag: 18.07.1996
(51) Int. Cl.: C07D 249/12

(54) **Verfahren zur Herstellung von substituierten Aminocarbonyltriazolinonen**
Process for the preparation of substituted aminocarbonyltriazolinones
Procédé pour la préparation d'aminocarbonyltriazolinones substituées

(30) Priorität: 31.07.1995 DE 19528055
(43) Veröffentlichungstag der Anmeldung: 05.02.1997
(73) Patentinhaber: BAYER AG, 51368 Leverkusen (DE)
(72) Erfinder: Diehr, Hans-Joachim, Dr., 42329 Wuppertal (DE)

(56) Entgegenhaltungen:
- EP-A- 0 294 666
- EP-A- 0 370 293
- CHEMICAL ABSTRACTS, vol. 122, no. 1, 2.Januar 1995 Columbus, Ohio, US; abstract no. 9682u, Seite 1079; XP002016161 & JP-A-06 228 080 (HODOGAYA CHEMICAL CO LTD) 16.August 1994

## Beschreibung

Die Erfindung betrifft ein neues Verfahren zur Herstellung von substituierten Aminocarbonyltriazolinonen, welche als herbizid wirksame Verbindungen bekannt sind.

Es ist bekannt, daß man bestimmte substituierte Aminocarbonyltriazolinone, wie z.B. die Verbindung 4-Amino-5-methyl-2-t-butylaminocarbonyl-2,4-dihydro-3H-1,2,4-triazol-3-on, erhält, wenn man entsprechende Triazolinone, wie z.B. die Verbindung 4-Amino-5-methyl-2,4-dihydro-3H-1,2,4-triazol-3-on mit Isocyanaten, wie z.B. t-Butylisocyanat, in Gegenwart eines Reaktionshilfsmittels, wie z.B. Diazabicycloundecen (DBU), und in Gegenwart eines Verdünnungsmittels, wie z.B. Acetonitril umsetzt. (vgl. EP 294666, vgl. auch EP 370293).

Bei dieser Herstellungsweise werden jedoch die gewünschten substituierten Aminocarbonyltriazolinone in erheblichem Umfang durch Produkte zweifacher Isocyanat-Addition verunreinigt und somit auch in unbefriedigenden Ausbeuten erhalten.

Für die Herstellung von substituierten Aminocarbonyltriazolinonen in befriedigender Qualität war es daher bisher notwendig, eine Schutzgruppe für die Aminogruppe zu verwenden und nach der Umsetzung mit einem Isocyanat die Schutzgruppe wieder abzuspalten. Diese dreistufige Reaktionsfolge führt zu Ausbeuteverlusten, insbesondere auch deswegen, weil unter den sauren Bedingungen, die zur Abspaltung der Schutzgruppe notwendig sind, auch die substituierten Aminocarbonyltriazolinone nicht ganz stabil sind.

Es wurde nun gefunden, daß man substituierte Aminocarbonyltriazolinone der allgemeinen Formel (I) 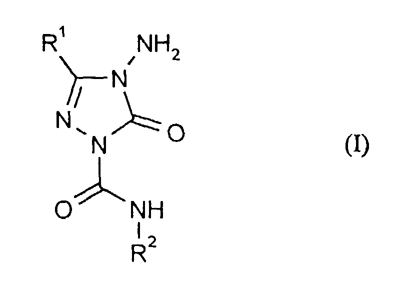 in welcher
- R¹: für einen jeweils gegebenenfalls substituierten Rest der Reihe Alkyl, Alkoxy, Alkylthio, Alkylamino oder Dialkylamino steht,
- R²: für einen jeweils gegebenenfalls substituierten Rest der Reihe Alkyl, Alkenyl, Alkinyl, Cycloalkyl, Cycloalkylalkyl, Arylalkyl, Arylalkenyl oder Arylalkinyl steht,
in sehr guten Ausbeuten und in hoher Reinheit erhält,
wenn man substituierte Triazolinone der allgemeinen Formel (II) 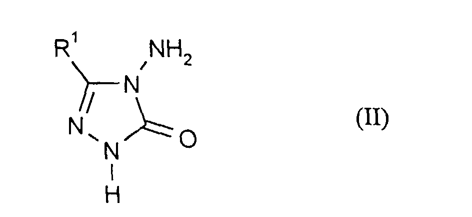 in welcher
- R¹: die oben angegebene Bedeutung hat,
mit Isocyanaten der allgemeinen Formel (III)

O=C=N-R² (III)

in welcher
- R²: die oben angegebene Bedeutung hat,
in Gegenwart eines Reaktionshilfsmittels ausgewählt aus Lithiumhydrid, Lithiumhydroxid, Lithiumfluorid, Lithiumchlorid, Lithiumbromid, Lithiumacetat, Lithiumcarbonat oder Lithiumsulfat und in Gegenwart einer basischen Verbindung sowie in Gegenwart eines Verdünnungsmittels bei Temperaturen zwischen 0°C und 150°C umsetzt.

Überraschenderweise können die substituierten Aminocarbonyltriazolinone der allgemeinen Formel (I) nach dem erfindungsgemäßen Verfahren in erheblich höheren Ausbeuten und in wesentlich besserer Qualität als nach dem bekannten Stand der Technik hergestellt werden.

Das erfindungsgemäße Verfahren stellt somit eine wertvolle Bereicherung des Standes der Technik dar.

Das erfindungsgemäße Verfahren betrifft vorzugsweise die Herstellung von Verbindungen der Formel (I), in welcher
- R¹: für einen jeweils gegebenenfalls durch Cyano, Halogen oder C₁-C₄-Alkoxy substituierten Rest der Reihe Alkyl, Alkoxy, Alkylthio, Alkylamino oder Dialkylamino mit jeweils 1 bis 6 Kohlenstoffatomen steht,
- R²: für einen jeweils gegebenenfalls durch Cyano oder Halogen substituierten Rest der Reihe Alkyl, Alkenyl, Alkinyl mit jeweils 1 bis 12 Kohlenstoffatomen, oder für einen jeweils gegebenenfalls durch Cyano, Halogen, C₁-C₄-Alkyl, C₁-C₄-Halogenalkyl, C₁-C₄-Alkoxy oder C₁-C₄-Halogenalkoxy substituierten Rest der Reihe C₃-C₆-Cycloalkyl, C₁-C₆-Cycloalkyl-C₁-C₄-alkyl, Phenyl-C₁-C₄-alkyl, Phenyl-C₂-C₄-alkenyl oder Phenyl-C₂-C₄-alkinyl steht.

Das erfindungsgemäße Verfahren betrifft insbesondere die Herstellung von Verbindungen der Formel (I), in welcher
- R¹: für jeweils gegebenenfalls durch Cyano, Fluor, Chlor, Brom, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t- Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino, Diethylamino oder Dipropylamino steht,
- R²: für jeweils gegebenenfalls durch Cyano, Fluor, Chlor oder Brom substituiertes Methyl, Ethyl, n- oder i-Propyl, n-, i-, s- oder t-Butyl, n-, i-, s- oder t-Pentyl, n-, i- oder s-Hexyl, Propenyl, Butenyl, Pentenyl, Propinyl, Butinyl oder Pentinyl, oder für jeweils gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Fluorethoxy, Difluorethoxy oder Trifluorethoxy substituiertes Cyclohexyl, Cyclohexylmethyl, Cyclohexylethyl, Cyclohexylpropyl, Benzyl, Phenylethyl, Phenylpropyl, Phenylethenyl, Phenylpropenyl, Phenylethinyl oder Phenylpropinyl steht.

Verwendet man beispielsweise 4-Amino-5-methyl-2,4-dihydro-3H-1,2,4-triazol-3-on und t-Butylisocyanat als Ausgangsstoffe, so kann der Reaktionsablauf beim erfindungsgemäßen Verfahren durch das folgende Formelschema skizziert werden:

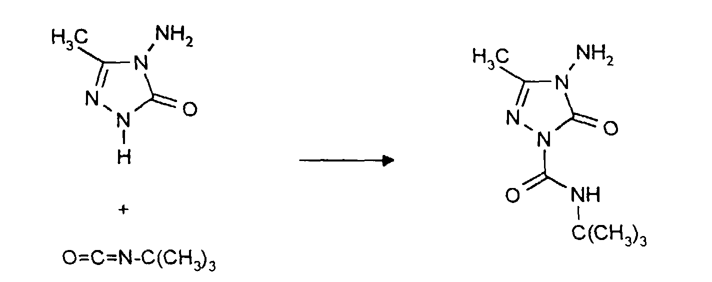

Die beim erfindungsgemäßen Verfahren zur Herstellung der Verbindungen der Formel (I) als Ausgangsstoffe zu verwendenden substituierten Triazolinone sind durch die Formel (II) allgemein definiert. In der Formel (II) hat R¹ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R¹ angegeben wurde.

Die Ausgangsstoffe der Formel (II) sind bekannt und/oder können nach bekannten Verfahren hergestellt werden (vgl. EP 294666, EP 370293).

Die beim erfindungsgemäßen Verfahren weiter als Ausgangsstoffe zu verwendenden Isocyanate sind durch die Formel (III) allgemein definiert. In der Formel (III) hat R² vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R2 angegeben wurde.

Die Ausgangsstoffe der Formel (III) sind bekannte Synthesechemikalien.

Das erfindungsgemäße Verfahren wird in Gegenwart eines Reaktionshilfsmittels durchgeführt. Als Reaktionshilfsmittel werden Lithium-Hydrid, Hydroxid, Acetat, Carbonat, Sulfat oder Halogenide eingesetzt. Lithiumhydrid, Lithiumhydroxid, Lithiumfluorid, Lithiumchlorid und Lithiumbromid seien als Reaktionshilfsmittel für das erfindungsgemäße Verfahren besonders hervorgehoben.

Das erfindungsgemäße Verfahren wird in Gegenwart einer basischen Verbindung durchgeführt. Als basische Verbindungen kommen in diesem Zusammenhang im allgemeinen die üblichen anorganischen oder organischen Basen oder Säureakzeptoren in Betracht. Hierzu gehören vorzugsweise Alkalimetall- oder Erdalkalimetallacetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

Natriumhydroxid, Kaliumhydroxid, 1,5-Diazabicyclo[4,3,0]-non-5-en (DBN), und 1,8 Diazabicyclo[5,4,0]-undec-7-en (DBU) werden als basische Verbindungen bei der Durchführung des erfindungsgemäßen Verfahren besonders bevorzugt eingesetzt.

Das erfindungsgemäße Verfahren wird in Gegenwart eines Verdünnungsmittels durchgeführt. Als Verdünnungsmittel kommen vorzugsweise aprotisch polare organische Lösungsmittel in Betracht. Hierzu gehören vorzugsweise Dialkylether, wie beispielsweise Diethylether, Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethylphosphorsäuretriamid; Ester, wie beispielsweise Essigsäure-methylester, -ethylester, -n- oder -i-propylester, -n-, -i- oder -s-butylester; sowie Sulfoxide, wie beispielsweise Dimethylsulfoxid.

Methyl-t-butylether, Methyl-t-pentylether, Tetrahydrofuran, Essigsäuremethylester, Essigsäureethylester und Acetonitril werden als Verdünnungsmittel beim erfindungsgemäßen Verfahren besonders bevorzugt eingesetzt.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 20°C und 110°C, insbesondere zwischen 40°C und 80°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens setzt man je Mol substituiertem Triazolinon der Formel (II) im allgemeinen zwischen 0,9 und 1,5 Mol, vorzugsweise zwischen 1,0 und 1,4 Mol, insbesondere zwischen 1,1 und 1,3 Mol Isocyanat der Formel (III), zwischen 0,001 und 1 Mol, vorzugsweise zwischen 0,01 und 0,1 Mol Reaktionshilfsmittel, sowie zwischen 0,001 und 1 Mol, vorzugsweise zwischen 0,01 und 0,1 Mol basische Verbindung ein.

In einer bevorzugten Ausführungsform des erfindungsgemäßen Verfahrens wird ein substituiertes Triazolinon der Formel (II) zusammen mit einem Reaktionshilfsmittel und einer basischen Verbindung in einem geeigneten Verdünnungsmittel vorgelegt und ein Isocyanat der Formel (III) wird langsam in diese Mischung eindosiert. Das Reaktionsgemisch wird dann gerührt, bis die Umsetzung praktisch abgeschlossen ist.

Die Aufarbeitung und Isolierung des Produktes der Formel (I) kann nach üblichen Methoden durchgeführt werden. Beispielsweise wird filtriert und vom Filtrat das Lösungsmittel unter vermindertem Druck sorgfältig abdestilliert, wobei das gewünschte Produkt im allgemeinen in guter Qualität als Rückstand verbleibt. Es kann bei Bedarf auf übliche Weise, beispielsweise durch Umkristallisieren weiter gereinigt werden.

Die nach dem erfindungsgemäßen Verfahren herzustellenden substiuierten Aminocarbonyltriazolinone der allgemeinen Formel (I) sind bereits als herbizid wirksame Verbindungen bekannt (vgl. EP 294666, EP 370293).

### Herstellungsbeispiele:

### Beispiel 1

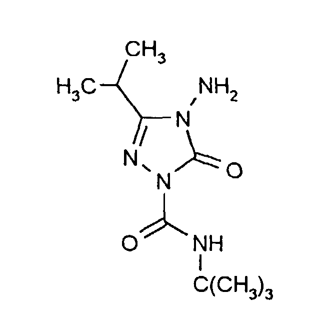

54,5 g (0,55 Mol) t-Butylisocyanat werden innerhalb von ca. 20 Minuten zu einer auf 55°C bis 60°C aufgeheizten Mischung aus 72,8 g (0,50 Mol) 4-Amino-5-i-propyl-2,4-dihydro-3H-1,2,4-triazol-3-on (97,6%ig), 0,8 g Kaliumhydroxid-Schuppen (88%ig), 0,5 g Lithiumchlorid und 500 ml Essigsäuremethylester tropfenweise unter Rühren gegeben und die Reaktionsmischung wird dann 3 Stunden unter Rückfluß erhitzt. Nach Abkühlen auf ca. 20°C wird filtriert, vom Filtrat das Lösungsmittel im Wasserstrahlvakuum sorgfältig abdestilliert und der verbleibende Rückstand bei ca. 60°C im Wasserstrahlvakuum getrocknet.

Man erhält 122 g (Gehalt: 93,2%, d.h. Ausbeute: 94% der Theorie) 4-Amino-5-i-propyl-2-t-butylaminocarbonyl-2,4-dihydro-3H-1,2,4-triazol-3-on vom Schmelzbereich 158°C bis 162°C.

### Beispiel 2

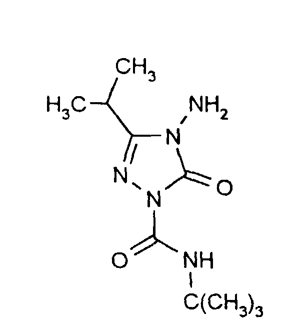

21,8 g (0,22 Mol) t-Butylisocyanat werden innerhalb von ca. 12 Minuten zu einer auf 55°C bis 60°C aufgeheizten Mischung aus 30,2 g (0,20 Mol) 4-Amino-5-i-propyl-2,4-dihydro-3H-1,2,4-triazol-3-on (94,2%ig), 0,32 g Kaliumhydroxid-Schuppen (88%ig), 0,20 g Lithiumchlorid und 200 ml Essigsäuremethylester tropfenweise unter Rühren gegeben und die Reaktionsmischung wird dann 3 Stunden unter Rückfluß erhitzt. Nach Abkühlen auf ca. 20°C wird vom Filtrat das Lösungsmittel im Wasserstrahlvakuum sorgfältig abdestilliert und der verbleibende Rückstand bei ca. 60°C im Wasserstrahlvakuum getrocknet.

Man erhält 51,8 g (Gehalt: 93,9%, d.h. Ausbeute: 96% der Theorie) 4-Amino-5-i-propyl-2-t-butylaminocarbonyl-2,4-dihydro-3H-1,2,4-triazol-3-on vom Schmelzbereich 158°C bis 162°C.

Bei Durchführung der Umsetzung analog Beispiel 2, jedoch ohne Einsatz von Lithiumchlorid wird die Verbindung 4-Amino-5-i-propyl-2-t-butylaminocarbonyl-2,4-dihydro-3H-1,2,4-triazol-3-on nur in einer Ausbeute von 73% der Theorie erhalten.

Analog Beispiel 1 und 2 sowie entsprechend der allgemeinen Beschreibung des erfindungsgemäßen Verfahrens können beispielsweise auch die in der nachstehenden Tabelle 1 aufgeführten Verbindungen der Formel (I) hergestellt werden.

**Tabelle 1:**

| Beispiele für die erfindungsgemäß herzustellenden Verbindungen | | | | |
|---|---|---|---|---|
| Bsp.-Nr. | R¹ | R² | Schmelzpunkt (°C) | Ausbeute (% d.Th.) |
| 3 | C₃H₇-i | C₄H₉-s | 68 | 100 |
| 4 | C₃H₇-i | C₃H₇-i | 132 | 100 |
| 5 | C₃H₇-i | C₄H₉-i | 84 | |
| 6 | C₃H₇-i | CH₃ | 174 | 98 |

## Patentansprüche

1. Verfahren zur Herstellung substituierter Aminocarbonyltriazolinone der allgemeinen Formel (I) 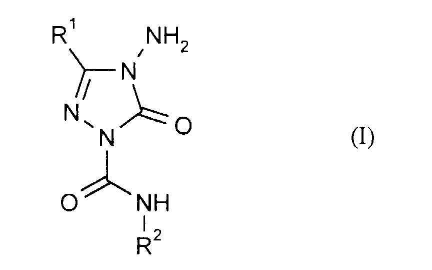 in welcher
R¹ für einen jeweils gegebenenfalls substituierten Rest der Reihe Alkyl, Alkoxy, Alkylthio, Alkylamino oder Dialkylamino steht,
R² für einen jeweils gegebenenfalls substituierten Rest der Reihe Alkyl, Alkenyl, Alkinyl, Cycloalkyl, Cycloalkylalkyl, Arylalkyl, Arylalkenyl oder Arylalkinyl steht,
dadurch gekennzeichnet, daß man substituierte Triazolinone der allgemeinen Formel (II) 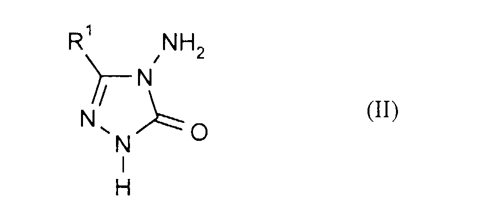 in welcher
R¹ die oben angegebene Bedeutung hat,
mit Isocyanaten der allgemeinen Formel (III)
O=C=N-R² (III)
in welcher
R² die oben angegebene Bedeutung hat,
in Gegenwart eines Reaktionshilfsmittels ausgewählt aus Lithiumhydrid. Lithiumhydroxid, Lithiumfluorid, Lithiumchlorid, Lithiumbromid. Lithiumacetat, Lithiumcarbonat oder Lithiumsulfat und in Gegenwart einer basischen Verbindung sowie in Gegenwart eines Verdünnungsmittels bei Temperaturen zwischen 0°C und 150°C umsetzt.

2. Verfahren gemäß Anspruch 1, dadurch gekennzeichnet, daß
R¹ für einen jeweils gegebenenfalls durch Cyano, Halogen oder C₁-C₄-Alkoxy substituierten Rest der Reihe Alkyl, Alkoxy, Alkylthio, Alkylamino oder Dialkylamino mit jeweils 1 bis 6 Kohlenstoffatomen steht,
R² für einen jeweils gegebenenfalls durch Cyano oder Halogen substituierten Rest der Reihe Alkyl, Alkenyl, Alkinyl mit jeweils 1 bis 12 Kohlenstoffatomen, oder für einen jeweils gegebenenfalls durch Cyano, Halogen, C₁-C₄-Alkyl, C₁-C₄-Halogenalkyl, C₁-C₄-Alkoxy oder C₁-C₄-Halogenalkoxy substituierten Rest der Reihe C₃-C₆-Cycloalkyl, C₃-C₆-Cycloalkyl-C₁-C₄-alkyl, Phenyl-C₁-C₄-alkyl, Phenyl-C₂-C₄-alkenyl oder Phenyl-C₂-C₄-alkinyl steht.

3. Verfahren gemäß Anspruch 1, dadurch gekennzeichnet, daß
R¹ für jeweils gegebenenfalls durch Cyano, Fluor, Chlor, Brom, Methoxy oder Ethoxy substituiertes Methyl, Ethyl, n- oder i-Propyl, n-, i-, s- oder t-Butyl, n-, i-, s- oder 1-Pentyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino, Diethylamino oder Dipropylamino steht,
R² für jeweils gegebenenfalls durch Cyano, Fluor, Chlor oder Brom substituiertes Methyl, Ethyl, n- oder i-Propyl, n-, i-, s- oder t-Butyl, n-, i-, s- oder t-Pentyl, n-, i- oder s-Hexyl, Propenyl, Butenyl, Pentenyl, Propinyl, Butinyl oder Pentinyl, oder für jeweils gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Fluorethoxy, Difluorethoxy oder Trifluorethoxy substituiertes Cyclohexyl, Cyclohexylmethyl, Cyclohexylethyl, Cyclohexylpropyl, Benzyl, Phenylethyl, Phenylpropyl, Phenylethenyl, Phenylpropenyl, Phenylethinyl oder Phenylpropinyl steht.

4. Verfahren gemäß einem der Ansprüche 1 bis 3, dadurch gekennzeichnet, daß die Umsetzung unter Normaldruck durchgeführt wird.

5. Verfahren gemäß einem der Ansprüche 1 bis 3, dadurch gekennzeichnet, daß pro Mol substituiertem Triazolinon der Formel (II) zwischen 0,9 und 1,5 Mol Isocyanat der Formel (III), zwischen 0,001 und 1 Mol Reaktionshilfsmittel sowie zwischen 0,001 und 1 Mol basische Verbindung eingesetzt werden.

6. Verfahren gemäß einem der Ansprüche 1 bis 3, dadurch gekennzeichnet, daß substituiertes Triazolinon der Formel (II) zusammen mit dem Reaktionshilfsmittel und der basischen Verbindung in einem geeigneten Verdünnungsmittel vorgelegt und Isocyanat der Formel (III) langsam in diese Mischung eindosiert wird.

## Claims

1. Process for the preparation of substituted aminocarbonyltriazolinones of the general formula (I) 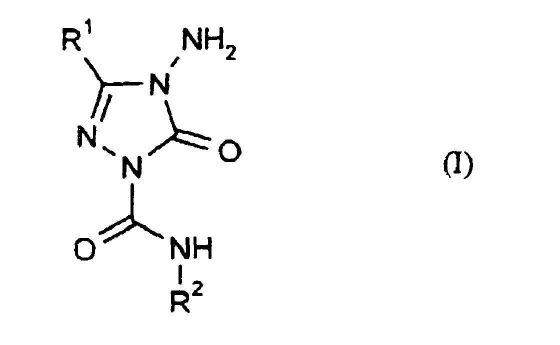 in which
R¹ represents a radical from the series consisting of alkyl, alkoxy, alkylthio, alkylamino and dialkylamino, each of which is optionally substituted, and
R² represents a radical from the series consisting of alkyl, alkenyl, alkinyl, cycloalkyl, cycloalkylalkyl, arylalkyl, arylalkenyl and arylalkinyl, each of which is optionally substituted,
characterized in that substituted triazolinones of the general formula (II) 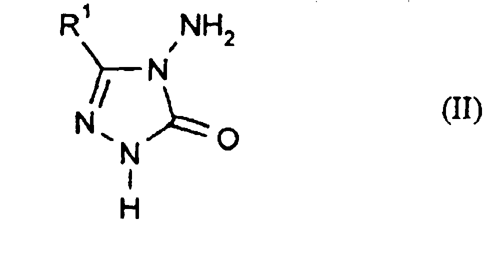 in which
R¹ has the abovementioned meaning
are reacted with isocyanates of the general formula (III)
O=C=N-R² (III)
in which
R² has the abovementioned meaning,
in the presence of a reaction auxiliary selected from the group consisting of lithium hydride, lithium hydroxide, lithium fluoride, lithium chloride, lithium bromide, lithium acetate, lithium carbonate or lithium sulphate and in the presence of a basic compound and in the presence of a diluent at temperatures of between 0°C and 150°C.

2. Process according to Claim 1, characterized in that
R¹ represents a radical from the series consisting of alkyl, alkoxy, alkylthio, alkylamino or dialkylamino, each of which has 1 to 6 carbon atoms and each of which is optionally substituted by cyano, halogen or C₁-C₄-alkoxy, and
R² represents a radical from the series consisting of alkyl, alkenyl and alkinyl, each of which has 1 to 12 carbon atoms and each of which is optionally substituted by cyano or halogen, or represents a radical from the series consisting of C₃-C₆-cycloalkyl, C₃-C₆-cycloalkyl-C₁-C₄-alkyl, phenyl-C₁-C₄-alkyl, phenyl-C₂-C₄-alkenyl or phenyl-C₂-C₄-alkinyl, each of which is optionally substituted by cyano, halogen, C₁-C₄-alkyl, C₁-C₄-halogenoalkyl, C₁-C₄-alkoxy or C₁-C₄-halogenoalkoxy.

3. Process according to Claim 1, characterized in that
R¹ represents methyl, ethyl, n- or i-propyl, n-, i-, s- or t-butyl, n-, i-, s- or t-pentyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino, diethylamino or dipropylamino, each of which is optionally substituted by cyano, fluorine, chlorine, bromine, methoxy or ethoxy, and
R² represents methyl, ethyl, n- or i-propyl, n-, i-, s- or t-butyl, n-, i-, s- or t-pentyl, n-, i- or s-hexyl, propenyl, butenyl, pentenyl, propinyl, butinyl or pentinyl, each of which is optionally substituted by cyano, fluorine, chlorine or bromine, or represents cyclohexyl, cyclohexylmethyl, cyclohexylethyl, cyclohexylpropyl, benzyl, phenylethyl, phenylpropyl, phenylethenyl, phenylpropenyl, phenylethinyl or phenylpropinyl, each of which is optionally substituted by cyano, fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy, trifluoromethoxy, fluoroethoxy, difluoroethoxy or trifluoroethoxy.

4. Process according to one of Claims 1 to 3, characterized in that the reaction is carried out under atmospheric pressure.

5. Process according to one of Claims 1 to 3, characterized in that between 0.9 and 1.5 mol of isocyanate of the formula (III), between 0.001 and 1 mol of reaction auxiliary and between 0.001 and 1 mol of basic compound are employed per mole of substituted triazolinone of the formula (II).

6. Process according to one of Claims 1 to 3, characterized in that substituted triazolinone of the formula (II) together with the reaction auxiliary and the basic compound are introduced into a suitable diluent and isocyanate of the formula (III) is slowly metered into this mixture.

## Revendications

1. Procédé de préparation d'aminocarbonyltriazolinones substituées de formule générale (I) : 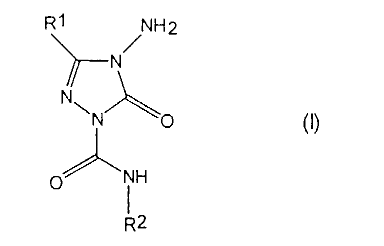
R¹représente un reste chaque fois facultativement substitué, de la série des alcoyle, alcoxy, alcoylthio, alcoylamino ou dialcoylamino ;
R² représente un reste chaque fois facultativement substitué, de la série des alcoyle, alcényle, alcynyle, cycloalcoyle, cycloalcoylalcoyle, arylalcoyle, arylalcényle ou arylalcynyle,
dans laquelle : caractérisé en ce que l'on fait réagir une triazolinone substituée de formule générale (II) : 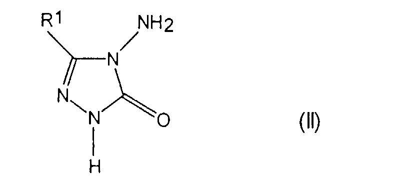 dans laquelle
R¹a la signification donnée ci-dessus, avec un isocyanate de formule générale (III) :
O=C=N-R² (III)
dans laquelle
R² a la signification donnée ci-dessus, en présence d'un auxiliaire de réaction choisi parmi l'hydrure de lithium, l'hydroxyde de lithium, le fluorure de lithium, le chlorure de lithium, le bromure de lithium, l'acétate de lithium, le carbonate de lithium ou le sulfate de lithium et en présence d'un composé basique ainsi qu'en présence d'un diluant, à des températures allant de 0°C à 150°C.

2. Procédé suivant la revendication 1, caractérisé en ce que
R¹ représente un reste chaque fois facultativement substitué par cyano, halogène ou alcoxy en C₁-C₄, de la série alcoyle, alcoxy, alcoylthio, alcoylamino ou dialcoylamino ayant chaque fois, 1 à 6 atomes de carbone ;
R² représente un reste chaque fois facultativement substitué par cyano ou halogène, de la série alcoyle, alcényle, alcynyle ayant chaque fois, 1 à 12 atomes de carbone, ou un reste chaque fois facultativement substitué par cyano, halogène, alcoyle en C₁-C₄, halogénoalcoyle en C₁-C₄, alcoxy en C₁-C₄ ou halogénoalcoxy en C₁-C₄, de la série cycloalcoyle en C₃-C₆, (cycloalcoyl en C₃-C₆)alcoyle en C₁-C₄, phénylalcoyle en C₁-C₄, phénylalcényle en C₂-C₄ ou phénylalcynyle en C₂-C₄.

3. Procédé suivant la revendication 1, caractérisé en ce que
R¹ représente méthyle, éthyle, n- ou i-propyle, n-, i-, s- ou t-butyle, n-, i-, s- ou t-pentyle, méthoxy, éthoxy, n- ou i-propoxy, n-, i-, s- ou t-butoxy, méthylthio, éthylthio, n- ou i-propylthio, n-, i-, s- ou t-butylthio, méthylamino, éthylamino, n- ou i-propylamino, n-, i-, s- ou t-butylamino, diméthylamino, diéthylamino ou dipropylamino, chaque fois facultativement substitué par cyano, fluor, chlore, brome, méthoxy ou éthoxy ;
R² représente méthyle, éthyle, n- ou i-propyle, n-, i-, s- ou t-butyle ou n-, i-, s- ou t-pentyle, n-, i- ou s-hexyle, propényle, butényle, pentényle, propynyle, butynyle ou pentynyle, chaque fois facultativement substitué par cyano, fluor, chlore ou brome, ou cyclohexyle, cyclohexylméthyle, cyclohexyléthyle, cyclohexylpropyle, benzyle, phényléthyle, phénylpropyle, phényléthényle, phénylpropényle, phényléthynyle ou phénylpropynyle, chaque fois facultativement substitué par cyano, fluor, chlore, brome, méthyle, éthyle, n- ou i-propyle, n-, i-, s- ou t-butyle, trifluorométhyle, méthoxy, éthoxy, n- ou i-propoxy, n-, i-, s- ou t-butoxy, difluorométhoxy, trifluorométhoxy, fluoroéthoxy, difluoroéthoxy ou trifluoroéthoxy.

4. Procédé suivant l'une quelconque des revendications 1 à 3, caractérisé en ce que la réaction est réalisée à pression normale.

5. Procédé suivant l'une quelconque des revendications 1 à 3, caractérisé en ce que par mole de triazolinone substituée de formule (II), on met en oeuvre entre 0,9 et 1,5 mole d'isocyanate de formule (III), entre 0,001 et 1 mole d'auxiliaire de réaction ainsi qu'entre 0,001 et 1 mole de composé basique.

6. Procédé suivant l'une quelconque des revendications 1 à 3, caractérisé en ce que l'on dispose la triazolinone substituée de formule (II) avec l'auxiliaire de réaction et le composé basique dans un diluant approprié et on ajoute lentement l'isocyanate de formule (III) à ce mélange.
